(19) Europäisches Patentamt
European Patent Office
Office européen des brevets

(11) **EP 2 581 738 B1**

(12) **EUROPEAN PATENT SPECIFICATION**

(45) Date of publication and mention
of the grant of the patent:
**14.01.2015 Bulletin 2015/03**

(51) Int Cl.:
***G01N 27/417*** *(2006.01)*      ***G01N 33/00*** *(2006.01)*

(21) Application number: **12187803.7**

(22) Date of filing: **09.10.2012**

(54) **Calibration technique for calibrating a zirconium oxide oxygen sensor and calibrated sensor**

Kalibriertechnik zur Kalibrierung eines Zirkonoxidsauerstoffsensors und kalibrierter Sensor

Technique d'étalonnage pour étalonner un capteur d'oxygène à oxyde de zirconium et capteur étalonné

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.10.2011 US 201161545372 P**

(43) Date of publication of application:
**17.04.2013 Bulletin 2013/16**

(73) Proprietor: **Mocon, Inc.**
**Minneapolis, Minnesota 55428 (US)**

(72) Inventor: **Howe, Michael D.**
**Blaine, MN 55449 (US)**

(74) Representative: **Vossius & Partner**
**Siebertstrasse 4**
**81675 München (DE)**

(56) References cited:
**US-A- 5 542 284      US-A1- 2005 139 468**

**Description**

**BACKGROUND**

Zirconium oxide oxygen sensors are widely used to continuously monitor the oxygen content of flue gases generated by such fuel-burning devices as boilers, kilns, ovens, internal combustion engines, driers, heat treating furnaces, incinerators, refinery process units, gas turbines, scrubbers and the like. Based on the information so gleaned, the mixture of oxygen and fuel may be adjusted to optimal levels. For example, the air being introduced into the combustion phase of a boiler may be regulated to achieve optimum efficiency, and to reduce nitrous oxide and/or sulphur dioxide emissions. Such monitoring systems can be employed to effectuate such adjustments continuously and automatically.

Zirconium oxide sensors are also employed in analytical equipment to precisely measure the oxygen content in sample gases. One such application is the $P_{AC}$ CHECK Benchtop $O_2$ Headspace Anayalzer available from Mocon, Inc. of Minneapolis, Minnesota.

Typically, zirconium oxide sensors consist of a ceramic tube made from zirconium oxide that has been stabilized with yttrium, with porous platinum electrodes coated opposite each other at the sensing end of the tube on the inner (monitoring) and outer (reference) surfaces of the tube. When the tube is heated to a temperature above about 600° C. (1100° F.) the ceramic material becomes permeable to oxygen ions, thus transforming the tube into an oxygen ion conducting solid electrolyte. When the number of oxygen molecules per unit volume is greater on one side of the tube relative to the other, oxygen ions migrate from the former to the latter. The platinum electrodes provide catalytic surfaces for the reduction of oxygen molecules into oxygen ions and oxidation of oxygen ions into oxygen molecules. Thus, oxygen molecules from the higher concentration side are reduced to oxygen ions at the electrode on the side of higher concentration and pass through the heated ceramic tube to the electrode on the side of lower concentration where they are oxidized back into oxygen molecules and released. This flow of ions creates an electron imbalance which produces a voltage potential between the electrodes. The magnitude of that potential is defmed by the "Nernst" equation as follows:

$$E=(RT/zF)LnQ$$

Where:

$E$ is the cell potential (electromotive force)
$R$ is the universal gas constant (8.314 472 J K$^{-1}$ mol$^{-1}$)
$T$ is the absolute temperature
$F$ is the Faraday constant (9.64853399×10$^4$ °C

mol$^{-1}$)
$z$ is the number of moles of electrons transferred in the cell reaction
$Q$ is the reaction quotient (*i.e.,* a function of the activities or concentrations of the chemical species involved in a chemical reaction).

The value of $z$ for zirconium oxide sensors is 4 as the redox reaction is:

$$O_2 +4e^- \leftrightarrow 2O^{-2}$$

The reaction quotient ($Q$) for zirconium oxide sensors is the ratio of the partial pressure of oxygen in the reference gas ($P1_{O2}$) to the partial pressure of oxygen in the monitored gas ($P2_{O2}$).

Substitution of these values into the Nernst equation produces the following equation for zirconium oxide sensors:

$$E=(RT/4F)Ln(P1_{O2}/P2_{O2})$$

Hence, the concentration of oxygen in a monitored gas ($P2_{O2}$) can be calculated from a voltage potential reading obtained from a zirconium oxide sensor at a known absolute temperature ($T$) so long as the concentration of oxygen in the reference gas ($P1_{O2}$) is known.

Due to slight variations in the performance of each zirconium oxide sensor, and variations for a given zirconium oxide sensor over time, Calibration Factors of Gain ($C_G$) (*i.e.,* deviation from ideal over the full output range) and Offset ($C_O$) (*i.e.,* deviation from ideal at minimum output) are typically established for each sensor and incorporated into the Nernst equation for zirconium oxide sensors to produce the following calibrated Nernst equation for zirconium oxide sensors:

$$E=(RT/4F)Ln(P1_{O2}/P2_{O2})C_G +C_O$$

The Calibration Factors of Gain ($C_G$) and Offset ($C_O$) are typically obtained for each sensor by taking readings from the sensor employing tank gases having known partial pressures of $O_2$ as the monitored gas ($P2_{O2}$), and air (which is 20.95% oxygen by volume) as the reference gas ($P2_{O1}$), and adjusting the values of Gain ($C_G$) and Offset ($C_O$) as necessary to cause the calculated value for $P2_{O2}$ obtained from each of the readings to match the known value for $P2_{O2}$ as closely as possible.

While effective for accurately calibrating zirconium oxide sensors, this calibration method is time consuming and expensive.

Accordingly, a substantial need exists for a low cost system and method for quickly, accurately and reliably calibrating zirconium oxide sensors.

## SUMMARY OF THE INVENTION

The invention involves the steps of (i) placing the first surface of the $ZrO_2$ sensor in fluid communication with a reference gas having a known non-zero concentration of oxygen at a known total first pressure, (ii) placing the second surface of the $ZrO_2$ sensor in fluid communication with the reference gas at a known total second pressure which is different than the known total first pressure to form a ∆P1 ∆P1 subjected zirconium oxide sensor, (iii) calculating an expected oxygen content reading from the ∆P1 subjected zirconium oxide sensor employing a calibrated Nernst equation for zirconium oxide sensors, (iv) taking an oxygen content reading with the ∆P1 subjected zirconium oxide sensor, (v) correlating the oxygen content reading taken with the ∆P1 subjected zirconium oxide sensor with the expected oxygen content reading for the ∆P1 subjected zirconium oxide sensor to create a correlated pair of ∆P1 ∆P1 values, and (vi) calibrating the zirconium oxide sensor employing the correlated pair of ∆P1 values.

## DETAILED DESCRIPTION OF A PREFERRED EMBODIMENT

### Definitions

As used herein, including the claims, the **"Calibrated Nernst equation for zirconium oxide sensors"** means:

$$E = (RT/4F)Ln(P1_{O2}/P2_{O2})C_G + C_O$$

Where:

E is the cell potential (electromotive force)
R is the universal gas constant (8.314 472 J K$^{-1}$ mol$^{-1}$)
T is the absolute temperature
F is the Faraday constant (9.64853399×10$^4$ °C mol$^{-1}$)
z is the number of moles of electrons transferred in the cell reaction (4)
$P1_{O2}$ is the partial pressure of oxygen in the reference gas
$P2_{O2}$ is the partial pressure of oxygen in the monitored gas
$C_G$ is the Gain Calibration Factor
$C_O$ is the Offset Calibration Factor.

and wherein at least one of the Calibration Factors of Gain ($C_G$) and Offset ($C_O$) are employed.

### Calibration Method

A reference gas having a known mole fraction of oxygen is selected. A preferred reference gas is air due to its ready availability and reliable static concentration of oxygen (*i.e.,* 20.86% assuming an RH of 50%). Tank gases containing a known non-zero concentration of oxygen may also be used as the reference gas.

A monitored gas having the same mole fraction of oxygen as the reference gas is selected. The monitored gas is preferably the same as the reference gas, and most preferrably obtained from the exact same source as the reference gas (*e.g.*, the immediately surrounding atmosphere).

The reference gas is placed into fluid communication with the exterior surface of the heated $ZrO_2$ ceramic tube at a known pressure, from which the partial pressure of oxygen in the reference gas $P1_{O2}$ can be calculated employing Dalton's Law of Partial Pressures since the mole fraction of oxygen in the reference gas is known. A preferred pressure for the reference gas is the current atmospheric pressure at the testing site.

The monitored gas is placed into fluid communication with the interior surface of the heated $ZrO_2$ ceramic tube at a known pressure which is different from the pressure of the reference gas in fluid communication with the exterior surface of the heated $ZrO_2$ ceramic tube. This can most conveniently be attained by introducing reference gas into the interior chamber of the $ZrO_2$ ceramic tube, sealing off the interior chamber, and then pulling a vacuum until the desired reduced pressure is attained. As with the reference gas, knowledge of the oxygen mole fraction and the total pressure of the monitored gas within the interior chamber of the $ZrO_2$ ceramic tube allows the partial pressure of oxygen in the monitored gas $P2_{O2}$ to be calculated employing Dalton's Law of Partial Pressures.

Readings are taken with the sensor at several different monitored gas pressures, with a corresponding calculated value for E, or alternatively converted to $O_2$ concentration, made using the Calibrated Nernst equation for zirconium oxide sensors for each reading, thereby creating a paired array of sensed and calculated values. Readings are preferably taken over a wide range of monitored gas pressures, with at least one and preferably a plurality of readings taken at a monitored gas pressure that is less than 50% of the reference gas pressure, most preferably less than 25% of the reference gas pressure and most preferably between 5% and 20% of the reference gas pressure.

The paired array of sensed and calculated values allow the Calibration Factors of Gain ($C_G$) and/or Offset ($C_O$) to be ascertained for the sensor employing standard calibration techniques well know to those of routine skill in the art.

## Claims

1. A method of calibrating a zirconium oxide sensor, comprising the steps of:

   (a) obtaining a zirconium oxide sensor operable for measuring oxygen content of a monitored gas by detecting passage of oxygen ions through a heated zirconium oxide ceramic partition having opposed first and second surfaces with the first surface in fluid communication with a reference gas having a known partial pressure of oxygen and the second surface in fluid communication with the monitored gas,
   (b) obtaining a first calibration value by (i) placing the first surface in fluid communication with a reference gas having a known non-zero concentration of oxygen at a known total first pressure, (ii) placing the second surface in fluid communication with the reference gas at a known total second pressure which is different than the known total first pressure to form a $\Delta P1$ subjected zirconium oxide sensor, and (iii) calculating an expected oxygen content reading from the $\Delta P1 \Delta P1$ subjected zirconium oxide sensor employing a calibrated Nernst equation for zirconium oxide sensors,
   (c) taking an oxygen content reading with the $\Delta P1$ subjected zirconium oxide sensor,
   (d) correlating the oxygen content reading taken with the $\Delta P1$ subjected zirconium oxide sensor with the expected oxygen content reading for the $\Delta P1 \Delta P1$ subjected zirconium oxide sensor to create a correlated pair of $\Delta P1 \Delta P1$ values, and
   (e) calibrating the zirconium oxide sensor employing the correlated pair of $\Delta P1 \Delta P1$ values.

2. The method of claim 1 further comprising the steps of:

   (f) obtaining a second calibration value by (i) placing the first surface in fluid communication with the reference gas at a known total first pressure, (ii) placing the second surface in fluid communication with the reference gas at a known total third pressure which is different than both the known total first and second pressures to form a $\Delta P2$ subjected zirconium oxide sensor, and (iii) calculating an expected oxygen content reading from the $\Delta P2$ subjected zirconium oxide sensor employing the calibrated Nernst equation for zirconium oxide sensors,
   (g) taking an oxygen content reading with the $\Delta P2$ subjected zirconium oxide sensor,
   (h) correlating the oxygen content reading taken with the $\Delta P2$ subjected zirconium oxide sensor with the expected oxygen content reading for the $\Delta P2$ subjected zirconium oxide sensor to create a correlated pair of $\Delta P2$ values, and
   (i) employing the correlated pair of $\Delta P2$ values along with the correlated pair of $\Delta P1 \Delta P1$ values to calibrate the zirconium oxide sensor.

3. The method according to any one of claims 1-2 wherein the reference gas and the monitored gas have the same mole fraction of oxygen.

4. The method according to any one of claims 1-3 wherein the reference gas and the monitored gas are obtained from the same source.

5. The method according to any one of claims 1-4 wherein the reference gas is air.

6. The method according to claim 5 wherein the air is obtained real-time from the surrounding free ambient air.

7. The method according to any one of claim 3 or 4 wherein the reference gas is employed at atmospheric pressure.

8. The method according to any one of claims 1 or 2 wherein the known total first pressure is atmospheric pressure.

9. The method according to any one of claims 3 or 4 wherein the monitored gas is employed at a total pressure of less than 20% of the total pressure at which the reference gas is employed.

10. The method according to any one of claims 1 or 2 wherein the known total second pressure is less than 20% of the known total first pressure.

11. The method according to claim 10 wherein the known total third pressure is at least twice the known total second pressure.

## Patentansprüche

1. Verfahren zur Kalibrierung eines Zirkonoxidsensors, das die Schritte aufweist:

   (a) Erhalten eines Zirkonoxidsensors, der so betreibbar ist, dass er den Sauerstoffgehalt eines überwachten Gases misst, indem er den Durchgang von Sauerstoffionen durch eine erwärmte Zirkonoxidkeramik-Trennwand mit einer ersten und einer entgegengesetzten zweiten Oberfläche detektiert, wobei die erste Oberfläche in Fluidverbindung mit einem Referenzgas steht, das einen bekannten Sauerstoffpartialdruck hat, und die zweite Oberfläche in Fluidverbindung mit dem überwachten Gas steht,

(b) Erhalten eines ersten Kalibrierwerts durch (i) Platzieren der ersten Oberfläche in Fluidverbindung mit einem Referenzgas, das eine bekannte von null abweichende Sauerstoffkonzentration bei einem bekannten ersten Gesamtdruck hat, (ii) Platzieren der zweiten Oberfläche in Fluidverbindung mit dem Referenzgas mit einem bekannten zweiten Gesamtdruck, der sich vom bekannten ersten Gesamtdruck unterscheidet, um einen ΔP1 unterliegenden Zirkonoxidsensor zu bilden, und (iii) Berechnen einer erwarteten Sauerstoffgehaltanzeige vom ΔP1 unterliegenden Zirkonoxidsensor unter Verwendung einer kalibrierten Nernst-Gleichung für Zirkonoxidsensoren,

(c) Ermitteln einer Sauerstoffgehaltanzeige mit dem ΔP1 unterliegenden Zirkonoxidsensor,

(d) Korrelieren der mit dem ΔP1 unterliegenden Zirkonoxidsensor ermittelten Sauerstoffgehaltanzeige mit der erwarteten Sauerstoffgehaltanzeige für den ΔP1 unterliegenden Zirkonoxidsensor, um ein korreliertes Paar ΔP1-Werte zu erzeugen, und

(e) Kalibrieren des Zirkonoxidsensors unter Verwendung des korrelierten Paars ΔP1-Werte.

2. Verfahren nach Anspruch 1, das ferner die Schritte aufweist:

(f) Erhalten eines zweiten Kalibrierwerts durch (i) Platzieren der ersten Oberfläche in Fluidverbindung mit dem Referenzgas mit einem bekannten ersten Gesamtdruck, (ii) Platzieren der zweiten Oberfläche in Fluidverbindung mit dem Referenzgas mit einem bekannten dritten Gesamtdruck, der sich vom bekannten ersten und zweiten Gesamtdruck unterscheidet, um einen ΔP2 unterliegenden Zirkonoxidsensor zu bilden, und (iii) Berechnen einer erwarteten Sauerstoffgehaltanzeige vom ΔP2 unterliegenden Zirkonoxidsensor unter Verwendung der kalibrierten Nernst-Gleichung für Zirkonoxidsensoren,

(g) Ermitteln einer Sauerstoffgehaltanzeige mit dem ΔP2 unterliegenden Zirkonoxidsensor,

(h) Korrelieren der mit dem ΔP2 unterliegenden Zirkonoxidsensor ermittelten Sauerstoffgehaltanzeige mit der erwarteten Sauerstoffgehaltanzeige für den ΔP2 unterliegenden Zirkonoxidsensor, um ein korreliertes Paar ΔP2-Werte zu erzeugen, und

(i) Verwenden des korrelierten Paars ΔP2-Werte zusammen mit dem korrelierten Paar ΔP1-Werte, um den Zirkonoxidsensor zu kalibrieren.

3. Verfahren nach Anspruch 1 oder 2, wobei das Referenzgas und das überwachte Gas die gleiche Molfraktion von Sauerstoff haben.

4. Verfahren nach einem der Ansprüche 1 bis 3, wobei das Referenzgas und das überwachte Gas aus derselben Quelle erhalten werden.

5. Verfahren nach einem der Ansprüche 1 bis 4, wobei das Referenzgas Luft ist.

6. Verfahren nach Anspruch 5, wobei die Luft aus der umliegenden freien Umgebungsluft in Echtzeit erhalten wird.

7. Verfahren nach Anspruch 3 oder 4, wobei das Referenzgas bei atmosphärischem Druck verwendet wird.

8. Verfahren nach Anspruch 1 oder 2, wobei der bekannte erste Gesamtdruck der atmosphärische Druck ist.

9. Verfahren nach Anspruch 3 oder 4, wobei das überwachte Gas bei einem Gesamtdruck unter 20 % des Gesamtdrucks verwendet wird, bei dem das Referenzgas verwendet wird.

10. Verfahren nach Anspruch 1 oder 2, wobei der bekannte zweite Gesamtdruck unter 20 % des bekannten ersten Gesamtdrucks liegt.

11. Verfahren nach Anspruch 10, wobei der bekannte dritte Gesamtdruck mindestens doppelt so groß wie der bekannte zweite Gesamtdruck ist.

**Revendications**

1. Procédé d'étalonnage d'un capteur à oxyde de zirconium, comprenant les étapes suivantes :

(a) l'obtention d'un capteur à oxyde de zirconium permettant de mesurer la teneur en oxygène d'un gaz surveillé par détection du passage d'ions oxygène au travers d'une cloison céramique en oxyde de zirconium chauffée comprenant une première et une seconde surface opposées, la première surface étant en communication fluidique avec un gaz de référence ayant une pression partielle d'oxygène connue et la seconde surface étant en communication fluidique avec le gaz surveillé,

(b) l'obtention d'une première valeur d'étalonnage par (i) mise en communication fluidique de la première surface avec un gaz de référence ayant une concentration d'oxygène non nulle connue à une première pression totale connue, (ii) mise en communication fluidique de la seconde surface avec le gaz de référence à une deuxième pression totale connue qui est différente de la première pression totale connue pour former un capteur à oxyde de zirconium soumis à ∆P1, et (iii) calcul d'une lecture de teneur en oxygène attendue à partir du capteur à oxyde de zirconium soumis à ∆P1 en utilisant une équation de Nernst étalonnée pour les capteurs à oxyde de zirconium,

(c) la réalisation d'une lecture de teneur en oxygène avec le capteur à oxyde de zirconium soumis à ∆P1,

(d) la corrélation de la lecture de teneur en oxygène réalisée avec le capteur à oxyde de zirconium soumis à ∆P1 avec la lecture de teneur en oxygène attendue pour le capteur à oxyde de zirconium soumis à ∆P1 afin de créer une paire corrélée de valeurs ∆P1, et

(e) l'étalonnage du capteur à oxyde de zirconium en utilisant la paire corrélée de valeurs ∆P1.

2. Procédé selon la revendication 1, comprenant en outre les étapes suivantes :

(f) l'obtention d'une seconde valeur d'étalonnage par (i) mise en communication fluidique de la première surface avec le gaz de référence à une première pression totale connue, (ii) mise en communication fluidique de la seconde surface avec le gaz de référence à une troisième pression totale connue qui est différente à la fois de la première et de la deuxième pression totale connue pour former un capteur à oxyde de zirconium soumis à ∆P2, et (iii) calcul d'une lecture de teneur en oxygène attendue à partir du capteur à oxyde de zirconium soumis à ∆P2 en utilisant l'équation de Nernst étalonnée pour les capteurs à oxyde de zirconium,

(g) la réalisation d'une lecture de teneur en oxygène avec le capteur à oxyde de zirconium soumis à ∆P2,

(h) la corrélation de la lecture de teneur en oxygène réalisée avec le capteur à oxyde de zirconium soumis à ∆P2 avec la lecture de teneur en oxygène attendue pour le capteur à oxyde de zirconium soumis à ∆P2 afin de créer une paire corrélée de valeurs ∆P2, et

(i) l'utilisation de la paire corrélée de valeurs ∆P2 et de la paire corrélée de valeurs ∆P1 pour étalonner le capteur à oxyde de zirconium.

3. Procédé selon l'une quelconque des revendications 1 à 2, dans lequel le gaz de référence et le gaz surveillé contiennent la même fraction molaire d'oxygène.

4. Procédé selon l'une quelconque des revendications 1 à 3, dans lequel le gaz de référence et le gaz surveillé sont obtenus à partir de la même source.

5. Procédé selon l'une quelconque des revendications 1 à 4, dans lequel le gaz de référence est l'air.

6. Procédé selon la revendication 5, dans lequel l'air est obtenu en temps réel à partir de l'air ambiant libre environnant.

7. Procédé selon l'une quelconque des revendications 3 ou 4, dans lequel le gaz de référence est utilisé à pression atmosphérique.

8. Procédé selon l'une quelconque des revendications 1 ou 2, dans lequel la première pression totale connue est la pression atmosphérique.

9. Procédé selon l'une quelconque des revendications 3 ou 4, dans lequel le gaz surveillé est utilisé à une pression totale de moins de 20 % de la pression totale à laquelle le gaz de référence est utilisé.

10. Procédé selon l'une quelconque des revendications 1 ou 2, dans lequel la deuxième pression totale connue est de moins de 20 % de la première pression totale connue.

11. Procédé selon la revendication 10, dans lequel la troisième pression totale connue est au moins le double de la deuxième pression totale connue.